# EUROPEAN PATENT SPECIFICATION

(11) **EP 1 621 149 B1**
(45) Date of publication and mention of the grant of the patent: **27.02.2008**
(21) Application number: 05254667.8
(22) Date of filing: 27.07.2005
(51) Int. Cl.: A61B 17/12

(54) **Embolic coil delivery system with u-shaped fiber release mechanism**
Einführvorrichtung für eine Emboliespirale mit U-förmigem Faserlösemechanismus
Dispositif de déploiement d'une spirale embolique avec une fibre de détachement en forme de U

(30) Priority: 30.07.2004 US 592901 P; 25.07.2005 US 188513 P
(43) Date of publication of application: 01.02.2006
(73) Proprietor: Cordis Neurovascular, Inc., Miami Lakes, Florida 33014 (US)
(72) Inventor: Sowers, William W., Pembroke Pines, Florida 33332 (US)
(74) Representative: Belcher, Simon James

(56) References cited:
- EP-A- 1 188 413
- US-A- 5 527 338
- US-A1- 2002 165 569

## Description

The present invention relates to a medical device for placing an embolic device such as an embolic coil, at a predetermined site within a vessel, and more particularly relates to a catheter based deployment system for delivering an embolic coil. This device is particularly suited to transport an embolic device, such as an embolic coil, through the tortious vasculature of the human brain and to the predetermined site within the vessel.

For many years, flexible catheters have been used to place various devices within the vasculature of the human body. Such devices include dilation balloons, radiopaque fluids, liquid medications, and various types of occlusion devices such as balloons and embolic coils. Examples of such catheter-based devices are disclosed in US- 5108407, entitled "Method and Apparatus for Placement of an Embolic Coil;" and US- 5122136, entitled "Endovascular Electrolytically Detachable Guidewire Tip for the Electroformation of Thrombus in Arteries, Veins, Aneurysms, Vascular Malformations and Arteriovenous Fistulas." These patents disclose catheter-based devices designed to deliver embolic coils to a predetermined site within a vessel of the human body in order to treat aneurysms, or alternatively, to occlude a blood vessel at a particular location.

Coils which are placed in vessels may take the form of helically wound coils, or alternatively, may take the form of randomly wound coils, coils wound within coils or other such coil configurations. Examples of various coil configurations are disclosed in US-5334210, entitled "Vascular Occlusion Assembly;" US-5382259, entitled, "Vasoocclusion Coil with Attached Tubular Woven or Braided Fibrous Covering, and EP1188413, entitled "Intravascular embolization device". Embolic coils are generally formed of a radiopaque metallic material, such as platinum, gold, tungsten, or an alloy of these metals. Often, several coils are placed at a given location to occlude the flow of blood through the vessel or aneurysm by promoting thrombus formation at the particular location.

Additionally, embolic coils have been placed within the distal end of a catheter, such that when the distal end of the catheter is properly positioned, the coil may then be pushed out of the end of the catheter with a pusher member to release the coil at the predetermined site within the vessel. This procedure for placement of the embolic coil is conducted under fluoroscopic visualization, such that the movement of a coil through the vasculature of the body may be monitored, and the coil may be placed in the desired location.

Another procedure involves the use of glue or solder to attach the coil to a guidewire, which is then placed within a flexible catheter for positioning the coil at a predetermined site within the vessel. Once the coil is at the predetermined site, the catheter holds the coil in position, and the guidewire is pulled proximally of the catheter to thereby detach the coil from the guidewire. Such a coil positioning system is disclosed in US-5263964 entitled, "Coaxial Traction Detachment Apparatus and Method."

Still another coil positioning procedure is that of having a catheter with a socket at the distal end, such that it retains a ball that is bonded to the proximal end of the coil. The ball, generally larger in diameter than the outside diameter of the coil, is placed in a socket within the lumen at the distal end of the catheter, and the catheter is then moved into a vessel in order to place the coil at a predetermined location. Once the site is reached, a pusher wire with a piston at the end thereof is pushed distally from the proximal end of the catheter to push the ball out of the socket, in order to release the coil at the predetermined site. Such a system is disclosed in US-5350397, entitled, "Axially Detachable Embolic Coil Assembly."

Another procedure for placing an embolic coil at a predetermined site within a vessel is that of using a heat releasable adhesive bond for retaining the coil at the distal end of the catheter. One such system uses laser energy transmitted through a fiber optic cable to apply heat to the adhesive bond in order to release the coil from the distal end of the catheter. Such a procedure is disclosed in aforementioned US-5108407.

Still another coil deployment system incorporates an interlocking mechanism with the coil. The interlocking end of the embolic coil couples with a similar interlocking end on a pusher assembly. A control wire extends through the two interlocking ends to secure the coil to the pusher assembly. The pusher assembly and embolic coil are initially disposed within the lumen of a catheter. When the embolic coil is pushed out of the end of the catheter for placement, the control wire is retracted and the coil disengages from the pusher assembly. Such a deployment system is disclosed in US-5925059, entitled, "Detachable Embolic Coil Assembly."

Yet another coil deployment system incorporates an embolic device detachably mounted on the distal portion of a pusher member and held in place with a connector thread or fiber. The fiber passes through a cutter member that may be activated to cut the connector fiber. Once the connector fiber is cut, the embolic device is released. Such a deployment system is disclosed in Published US-A-2002/0165569, and entitled, "Intravascular Device Deployment Mechanism Incorporating Mechanical Detachment."

Still another coil deployment system incorporates an embolic device with a stretch resistant member threrethrough. The distal end of the stretch resistant member is attached to the embolic coil, and the proximal end of the stretch resistant member is detachably mounted on an elongated pusher member to allow for placement and release of the coil within a vessel. The stretch resistant member is detachably mounted on the pusher member through various means, such as adhesive or by a connector fiber adhered to or tied onto the pusher member and is detachable by the application of heat. Such a deployment system is disclosed in Published US-A-2004/0034363, entitled, "Stretch Resistant Therapeutic Device."

Still another coil deployment system incorporates a platinum wire and or tip that is inserted into a vascular cavity. The tip may be elongated and flexible, folded upon itself several times, or may have a branched configuration. The tip may be separated from the wire mechanically or via electrolytic separation. Such a system is disclosed in US-5540680; US-5895385; US-5925037; and US-5976126, all entitled, "Endovascular Electrolytically Detachable Wire and Tip for the Formation of Thrombus in Arteries, Veins, Aneurysms, Vascular Malformations, and Arteriovenous Fistulas."

Still another coil deployment system incorporates a pusher member, having a stiff wavy-shaped wire end segment, coupled to an embolic coil and placed within the lumen of the catheter. The coil is advanced through the catheter until it reaches the predetermined site within the vessel, at which time the pusher member is retracted and the embolic coil is released. Such a system is disclosed in US-6203547, entitled, "Vaso-occlusion Apparatus Having a Manipulable Mechanical Detachment Joint and a Method for Using the Apparatus."

Still another embolic device deployment system includes an elongated flexible pusher member slidably disposed within a lumen of a catheter. An embolic device is retained at the end of the pusher member with a detachment filament. When the embolic device is advanced to the predetermined site within the vessel, the detachment filament is withdrawn releasing the embolic device. Such a system is disclosed in US-A-2006/0025801 filed on June 3, 2005, entitled, "Embolic Device Deployment System with Filament Release."

The present invention is directed toward a vasooclusive embolic device deployment system for use in placing an embolic device at a predetermined site within a vessel including an elongated flexible catheter and an elongated pusher member slidably disposed within the lumen of the catheter. Disposed at the distal end of the pusher member is an embolic device, preferably having a headpiece with an aperture therethrough coupled to its proximal end. Alternately, the aperture through the embolic device, which may take the form of a helically wound embolic coil, is formed by bending one of the helical turns at an angle to the remainder of the turns or by soldering a loop to the proximal end of the embolic device.

In accordance with an aspect of the present invention, a detachment fiber includes a U-shaped distal section, preferably constructed from platinum, which is sufficiently stiff to maintain a pre-shaped configuration. When the fiber is pulled proximally, it returns to a generally straight configuration. The detachment fiber extends from a position proximal of the proximal end of the device through the lumen of the catheter and toward the embolic device. The U-shaped distal section engages the aperture through the headpiece of the embolic device, such that when the fiber is pulled proximally the U-shaped distal section straightens to thereby release the embolic device.

In accordance with another aspect of the present invention, the pusher member includes a lumen therethrough and the embolic device is slidably disposed within the distal end of the lumen of the pusher member. A projection extends inwardly from a wall of the lumen of the pusher member at a position proximal of the embolic device to prevent the embolic device from sliding proximally into the lumen of the pusher.

In accordance with yet another aspect of the present invention, the detachment fiber extends through the lumen of the pusher member toward the embolic device. The U-shaped distal section engages the embolic device disposed within the lumen of the distal end of the pusher member.

In accordance with still another aspect of the present invention, a releasable clamp, having a lumen extending therethrough is mounted on the proximal end of the pusher member. The detachment fiber extends through the lumen of the clamp, so that upon release of the clamp the detachment fiber may be pulled proximally to release the embolic device.

Embodiments of the invention will now be described by way of example with reference to the accompanying drawings, in which:
Figure 1 is an enlarged, partially sectional view of an embodiment of an embolic device deployment system in accordance with the present invention; and,
Figures 2a, 2b, and 2c are enlarged, partially sectional views of the distal end of the coil deployment system shown in Figure 1, illustrating the sequential steps in the advancement of the embolic device, removal of a detachment fiber, and release of the embolic device.

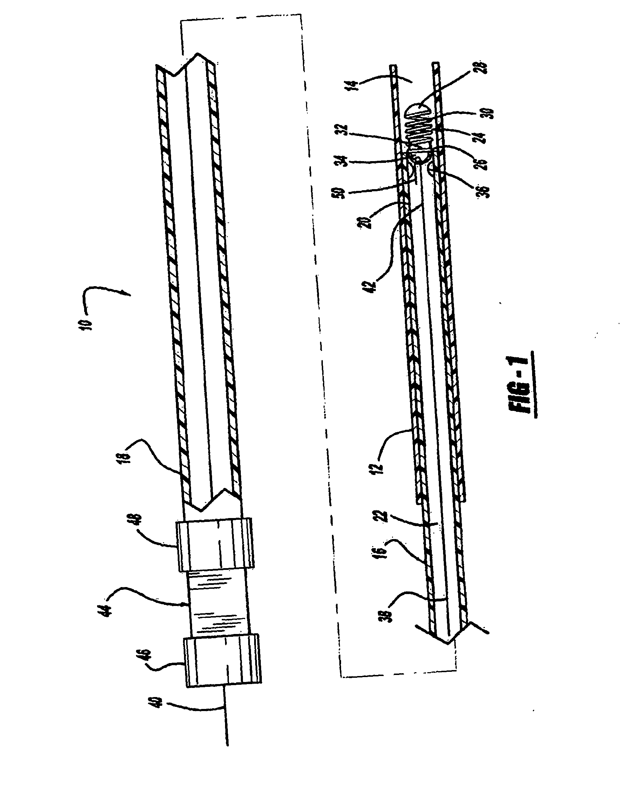

Figure 1 generally illustrates one embodiment of a U-shaped detachment fiber arrangement of an embolic device deployment system 10 of the present invention including an elongated flexible catheter 12 having a lumen 14 therethrough. An elongated flexible pusher member 16 having a proximal end 18, a distal end 20, and preferably having a lumen extending therethrough 22, is slidably disposed within the lumen 14 of the catheter 12. The pusher member 16 is constructed from a nickel titanium shape memory alloy, such as nitinol, but alternatively, may be constructed from any flexible, biocompatible material such as stainless steel, nylon, PTFE, other flexible materials, polymers, or composites.

An embolic device 24, having a proximal end 26 and a distal end 28, preferably taking the form of an embolic coil having a plurality of helical turns 30, is disposed within the lumen 22 of the distal end 20 of the pusher member 16. The embolic device 24 has a headpiece 32 coupled to its proximal end 26, the headpiece having an aperture 34 therethrough. The aperture 34 may alternately be constructed by bending one of the plurality of helical turns 30 at an angle to the remaining turns, or by welding an additional loop onto the embolic device 24 at an angle to the plurality of helical turns 30. Alternatively, the embolic device may take the form of embolic filaments, braids, expandable meshes, foams, and stents.

In addition, projections 36 are mounted on the wall of the lumen 22 of the pusher member 16 at a position proximal of the embolic device 24, to prevent the embolic device from moving proximally. Preferably, the projections 36 are constructed from platinum and secured with adhesive or, alternately, fused into the wall of the lumen 22 of the pusher member 16. Additionally, the embolic device 24 is further secured by a detachment fiber 38, having a proximal section 40 and a distal section 42 and preferably constructed from a light gauge metal wire such as platinum, nitinol, or other malleable materials. A releasable clamp 44, preferably taking the form of a Tuohy-Borst connector, having a proximal end 46 and a distal end 48 and a lumen therethrough, is mounted on the proximal end 18 of the pusher member 16 and secures the fiber 38.

The proximal section 40 of the detachment fiber 38 extends from a position proximal of the proximal end of the clamp 44 and through the lumen of the clamp. Subsequently, the fiber 38 extends through the lumen 22 of the pusher member 16 from its proximal end 18 toward its distal end 20, and releasably engages the embolic device 24 disposed within the lumen 22 of the distal end 20 of the pusher member 16. The distal section 42 of the detachment fiber 38, which extends through the aperture 34 through the headpiece 32 of the embolic device 24, is pre-shaped into a generally U-shaped configuration 50.

In order to prevent the fiber 38 from prematurely disengaging from the embolic device 24, the clamp 44 maintains tension on the fiber 38. The clamp 44 may be loosened to allow a proximal force to be applied to the proximal section 40 of the detachment fiber 38, to disengage the fiber 38 from the aperture 34 through the headpiece 32 of the embolic device 24, thus releasing the embolic device at the predetermined site within the vessel.

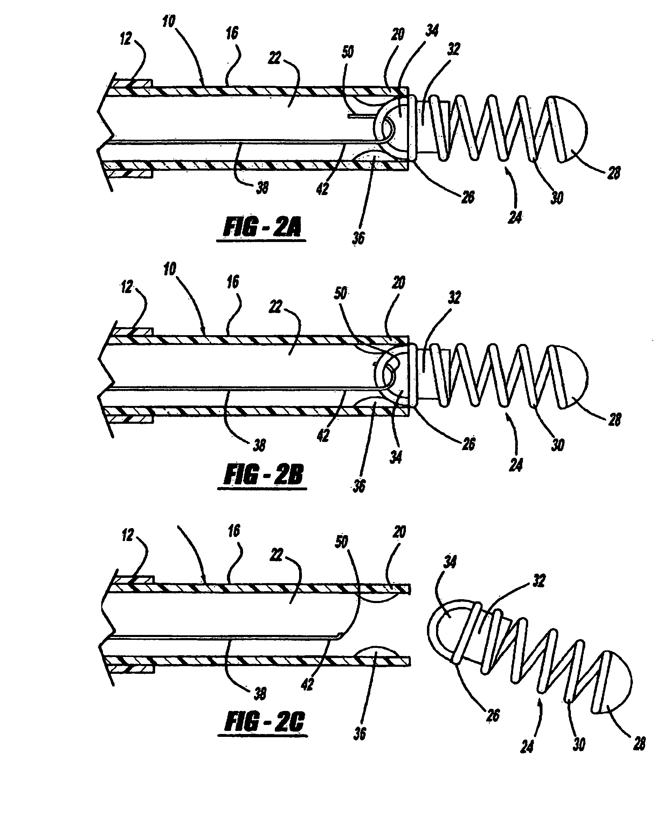

Figures 2a, 2b, and 2c generally illustrate the operation of the embolic device deployment system 10 and demonstrate the U-shaped detachment fiber release mechanism. More particularly, Figure 2a illustrates the distal end of the embolic device deployment system 10 after the pusher member 16 is advanced through the catheter 12, and has reached the predetermined site within the vessel. The detachment fiber 38 extends through the lumen 22 of the pusher member 16 toward the distal end 20. At the distal end 20 of the pusher member 16, the distal section 42 of the detachment fiber 38 extends through the aperture 34 through the headpiece 32 of the embolic device 24 in the U-shaped configuration 50. The U-shaped configuration 50 is formed of a material which exhibits the characteristic of being sufficiently stiff to maintain its pre-shaped, U-shaped configuration until pulled proximally from its proximal section to thereby straighten the U-shaped configuration 50. In order to increase the overall flexibility of the deployment system 10, the portion of the detachment fiber 38 proximal of the U-shaped configuration 50 may be constructed from a variable stiffness material created by tapering the fiber 38 from the proximal section 40 to the distal section 42.

Figure 2b illustrates the embolic device deployment system 10 after a proximal force has been applied to the detachment fiber 38, such that the detachment fiber 38 is partially disengaged from the aperture 34 through the headpiece 32 of the embolic device 24. The U-shaped configuration 50 straightens as the proximal force is applied. Figure 2c illustrates the embolic device deployment system 10, as the detachment fiber 38 is pulled further proximally. The U-shaped configuration 50 further deforms and straightens to release the embolic device 24 at the predetermined treatment site within the vessel.

One of the important advantages of the present invention is that the embolic device may be placed at a desired location within a vessel, or within an aneurysm, with the configuration of the device deployment system as shown in Figure 2a. If it is determined that the embolic device is improperly positioned, the embolic device 24 may then be withdrawn from that location and placed at another location, or even removed from the body by first withdrawing the pusher member 16 and the embolic device totally back into the catheter. Once the embolic device has been entirely withdrawn back into the delivery catheter, the catheter may then be moved to a more desirable location and the embolic device may then be released at the new location.

As is apparent, there are numerous modifications of the preferred embodiment described above which will be readily apparent to one skilled in the art, such as many variations and modifications of the embolic device including numerous coil winding configurations, or alternately other types of implant devices. There are variations in the material and configuration of the distal section of the detachment fiber as well as variations in the material and flexibility of the proximal portion of the detachment fiber. Additionally, there could be variations in the connector or in the method in which the detachment fiber is retained. These modifications would be apparent to those having ordinary skill in the art to which this invention relates and are intended to be within the scope of the claims which follow.

## Claims

1. A vasooclusive embolic device deployment system (10) for use in placing an embolic device at a predetermined site within a vessel comprising:
an elongated flexible catheter (12) having proximal and distal ends and a lumen (14) extending therethrough;
an elongated pusher member (16) having proximal (18) and distal (20) ends and a lumen (22) extending therethrough and being slidably disposed within the lumen(14) of the catheter(12);
an embolic device (24) having proximal(26) and distil (28) ends and being slidably disposed within the distal end (20) of the pusher member (16) ; and,
a detachment fiber (38) having a distal section(42) which exhibits the characteristics of maintaining a pre-shaped configuration until an external pulling force is applied to the fiber, said detachment fiber (38) extending through said lumen (22) of said elongated pusher member (16) and having a distal section (42) which is pre-formed into a U-shaped configuration, said U-shaped distal section is releasably coupled to said embolic device (24), such that when the fiber is pulled proximally the U-shaped distal section straightens to release the embolic device (24).

2. A vasooclusive embolic device deployment system (10) as defined in Claim 1, wherein said embolic device (24) includes a headpiece (32) having an aperture (34) therethrough and being coupled to the proximal end (26) of said embolic device,(24), and the detachment fiber(38) extends from a position proximal of the proximal end (18) of the pusher member (16), through the lumen (22) of the pusher member (16), and the U-shaped distal section extends through the aperture (34) in the headpiece (32) of the embolic device (24) to thereby couple the detachment fiber (38) to the embolic device (24).

3. A vasooclusive embolic device deployment system (10) as defined in Claim 1, wherein a releasable clamp (44) having a lumen extending therethrough is mounted on the proximal end of the pusher member (16), said detachment fiber (38) extending through the lumen of the clamp (44) so that upon release of the clamp (44) the detachment fiber (38) may be pulled proximally to release the embolic device (24).

4. A vasooclusive embolic device deployment system (10) as defined in Claim 1, wherein a projection (36) extends inwardly from a wall of the lumen (22) of the pusher member (16) at a position proximal of the embolic device (24) to prevent the embolic device (24) from moving proximally.

5. A vasooclusive embolic device deployment system (10) as defined in Claim 1, wherein the distal section (42) of the fiber (38) is comprised of platinum.

6. A vasooclusive embolic device deployment system (10) as defined in Claim 1, wherein the detachment fiber (38) is comprised of a variable stiffness material.

## Patentansprüche

1. Einsatzsystem (10) mit einer gefäßverschließenden, embolischen Vorrichtung zur Verwendung beim Anordnen einer emoblischen Vorrichtung an einem vorbestimmten Ort innerhalb eines Gefäßes, das aufweist:
einen länglichen flexiblen Katheter (12) mit einem proximalen und einem distalen Ende und einem Lumen (14), das sich durch diesen erstreckt;
ein längliches Schubelement (16) mit einem proximalen (18) und einem distalen (20) Ende und einem Lumen (22), das sich durch dieses erstreckt und das verschieblich innerhalb des Lumens (14) des Katheters (12) angeordnet ist;
eine embolische Vorrichtung (24) mit einem proximalen (26) und einem distalen (28) Ende, die verschieblich innerhalb des distalen Endes (20) des Schubelementes (16) angeordnet ist; und
eine Freigabefaser (38), die einen distalen Abschnitt (42) hat, welcher die Eigenschaften des Beibehaltens einer vorgeformten Ausgestaltung zeigt, bis eine externe Zugkraft auf die Feder aufgebracht wird, wobei die Freigabefaser (38) sich durch das Lumen (22) des länglichen Schubelementes (16) erstreckt und einen distalen Abschnitt (42) hat, der in eine U-förmige Ausgestaltung vorgeformt ist, wobei der U-förmige distale Abschnitt freigebbar an die embolische Vorrichtung (24) gekoppelt ist, so daß, wenn die Faser proximal gezogen wird, der U-förmige distale Abschnitt sich geradelegt, um die embolische Vorrichtung (24) freizugeben.

2. Einsatzsystem (10) mit einer gefäßverschließenden, embolischen Vorrichtung nach Anspruch 1, bei dem die embolische Vorrichtung (24) ein Kopfstück (32) mit einer Öffnung (34) durch dieses umfaßt und an das proximale Ende (26) der embolischen Vorrichtung (24) gekoppelt ist, und bei dem die Freigabefaser (38) sich von einer Position proximal von dem proximalen Ende (18) des Schubelementes (16) durch das Lumen (22) des Schubelementes (16) erstreckt und der U-förmige distale Abschnitt sich durch die Öffnung (34) in dem Handstück (32) der embolischen Vorrichtung (24) erstreckt, um somit die Freigabefaser (38) an die embolische Vorrichtung (24) zu koppeln.

3. Einsatzsystem (10) mit einer gefäßverschließenden, embolischen Vorrichtung, nach Anspruch 1, wobei eine lösbare Klemme (44) mit einem Lumen, das sich durch diese erstreckt, auf dem proximalen Ende des Schubelementes (16) angeordnet ist, wobei sich die Freigabefaser (38) durch das Lumen der Klemme (44) erstreckt, so daß beim Freigeben der Klemme (44) die Freigabefaser (38) proximal gezogen werden kann, um die embolische Vorrichtung (24) freizugeben.

4. Einsatzsystem (10) mit einer gefäßverschließenden, embolischen Vorrichtung nach Anspruch 1, bei dem sich ein Vorsprung nach innen von einer Wand des Lumens (22) des Schubelementes (16) an einer Position proximal der embolischen Vorrichtung (24) erstreckt, um zu verhindern, daß sich die embolische Vorrichtung (24) proximal bewegt.

5. Einsatzsystem (10) mit einer gefäßverschließenden embolischen Vorrichtung nach Anspruch 1, bei dem der distale Abschnitt (42) der Faser (38) aus Platin besteht.

6. Einsatzsystem (10) mit einer gefäßverschließenden embolischen Vorrichtung nach Anspruch 1, bei dem die Freigabefaser (38) aus einem Material mit variabler Steifigkeit besteht.

## Revendications

1. Système de déploiement de dispositif embolique vaso-occlusif (10) destiné à être utilisé pour placer un dispositif embolique au niveau d'un site prédéterminé dans un vaisseau, comprenant :
◆ un cathéter souple allongé (12) ayant des extrémités proximale et distale et une lumière (14) s'étendant à travers celui-ci ;
◆ un élément poussoir allongé (16) ayant des extrémités proximale (18) et distale (20) et une lumière (22) s'étendant à travers celui-ci et étant disposé de manière coulissante dans la lumière (14) du cathéter (12),
◆ un dispositif embolique (24) ayant des extrémités proximale (26) et distale (28) et étant disposé de manière coulissante dans l'extrémité distale (20) de l'élément poussoir (16) ; et
◆ une fibre de détachement (38) ayant une section distale (42) qui laisse apparaître les caractéristiques de maintien d'une configuration préformée jusqu'à ce qu'une force de traction externe soit appliquée sur la fibre, ladite fibre de détachement (38) s'étendant à travers ladite lumière (22) dudit élément poussoir allongé (16) et ayant une section distale (42) qui est préformée dans une configuration en forme de U, ladite section distale en forme de U est couplée de manière amovible audit dispositif embolique (24), de sorte que lorsque la fibre est tirée de manière proximale, la section distale en forme de U se redresse pour libérer le dispositif embolique (24).

2. Système de déploiement de dispositif embolique vaso-occlusif (10) selon la revendication 1, dans lequel ledit dispositif embolique (24) comprend un embout (32) ayant une ouverture (34) à l'intérieur celui-ci et étant couplé à l'extrémité proximale (26) dudit dispositif embolique (24) et la fibre de détachement (38) s'étend à partir d'une position proximale de l'extrémité proximale (18) de l'élément poussoir (16), en passant par la lumière (22) de l'élément poussoir (16) et la section distale en forme de U s'étend à travers l'ouverture (34) dans l'embout (32) du dispositif embolique (24) pour coupler ainsi la fibre de détachement (38) au dispositif embolique (24).

3. Système de déploiement de dispositif embolique vaso-occlusif (10) selon la revendication 1, dans lequel un dispositif de serrage amovible (44) ayant une lumière s'étendant à travers celui-ci, est monté sur l'extrémité proximale de l'élément poussoir (16), ladite fibre de détachement (38) s'étendant à travers la lumière du dispositif de serrage (44) de sorte que suite au déblocage du dispositif de serrage (44), la fibre de détachement (38) peut être tirée de manière proximale pour libérer le dispositif embolique (24) .

4. Système de déploiement du dispositif embolique vaso-occlusif (10) selon la revendication 1, dans lequel une saillie (36) s'étend vers l'intérieur à partir d'une paroi de la lumière (22) de l'élément poussoir (16) à une position proximale du dispositif embolique (24) pour empêcher le dispositif embolique (24) de se déplacer de manière proximale.

5. Système de déploiement du dispositif embolique vaso-occlusif (10) selon la revendication 1, dans lequel la section distale (42) de la fibre (38) est composée de platine.

6. Système de déploiement de dispositif embolique vaso-occlusif (10) selon la revendication 1, dans lequel là fibre de détachement (38) est composée d'un matériau à rigidité variable.
